# FASCICULE DE BREVET EUROPEEN

(11) **EP 0 951 246 B1**
(45) Date de publication et mention de la délivrance du brevet: **07.05.2003**
(21) Numéro de dépôt: 97945916.1
(22) Date de dépôt: 13.11.1997
(51) Int. Cl.: A61B 17/70

(54) **SYSTEME D'OSTEOSYNTHESE A DEFORMATION ELASTIQUE POUR COLONNE VERTEBRALE**
ELASTISCH VERFORMBARES OSTEOSYNTHESESYSTEM FÜR DIE WIRBELSÄULE
OSTEOSYNTHESIS SYSTEM WITH ELASTIC DEFORMATION FOR SPINAL COLUMN

(30) Priorité: 15.11.1996 FR 9613956
(43) Date de publication de la demande: 27.10.1999
(73) Titulaire: Stryker France S.A., 93290 Tremblay-en-France (FR); Elberg, Jean François, 75017 Paris (FR)
(72) Inventeur: ELBERG, Jean-François, F-75017 Paris (FR)
(74) Mandataire: Le Forestier, Eric
(86) Numéro de dépôt international: FR9702037
(87) Numéro de publication internationale: WO98022033

(56) Documents cités:
- EP-A- 0 667 127
- WO-A-94/21185
- FR-A- 2 717 370
- FR-A- 2 718 946

## Description

L'invention concerne les systèmes d'ostéosynthèse pour colonne vertébrale.

On connaît d'après le document FR-2 659 546 un système d'ostéosynthèse pour colonne vertébrale ou rachis, qui comporte des organes d'ancrage destinés à être fixés à une série de vertèbres de la colonne, et au moins un élément de liaison rigide destiné à relier les organes d'ancrage entre eux. Après que le système a été fixé à une série de vertèbres et assemblé, il constitue un dispositif rigide immobilisant ces vertèbres entre elles. Cette rigidité permet au dispositif d'encaisser tout ou partie des contraintes à la place de la série de vertèbres concernée. Toutefois, la première vertèbre adjacente à la série est totalement libre de se déplacer par rapport à la série de vertèbres rigidifiée. Il peut alors apparaître entre la dernière vertèbre de la série et la première vertèbre libre une discontinuité brutale dans la répartition des contraintes le long de la colonne. Par conséquent, le disque interposé entre ces deux vertèbres est très sollicité, et on constate même une augmentation anormale des contraintes au niveau du disque. Il s'ensuit très souvent une accélération de la dégénérescence de ce disque. On parle alors d'un syndrome de néo-charnière.

Le document WO 94/21185 présente un dispositif d'ostéosynthèse pour colonne vertébrale dans lequel deux vis pédiculaires sont reliées par un organe de liaison ayant une portion centrale flexible en forme de "U" et décalée latéralement. Ainsi, lorsque les deux vertèbres sont soumises à des contraintes tendant à déplacer relativement les deux organes d'ancrage en rotation autour d'un axe de déformation, l'organe de liaison est soumis à un moment de flexion et se fléchit élastiquement autour de l'axe de déformation. Il encaisse donc partiellement ces contraintes. Le système recrée partiellement la biomécanique de l'unité fonctionnelle composée des deux vertèbres fixées ainsi que du disque intervertébral. Toutefois, ce dispositif a pour inconvénient que la possibilité de la rotation relative entre les deux vis pédiculaires est essentiellement fonction de l'élasticité de la portion intermédiaire. Autrement dit, c'est sur cette élasticité que repose l'aptitude de la portion intermédiaire à se déformer. Or, lorsque cette élasticité est élevée afin de limiter les déplacements relatifs, les vertèbres peuvent être amenées à subir d'importantes sollicitations qui peuvent leur être nuisibles. A l'inverse, lorsque l'élasticité est basse, le maintien des vertèbres est médiocre.

Un but de l'invention est de fournir un système d'ostéosynthèse permettant de contrôler l'aptitude de la portion intermédiaire à se déformer sans limiter le choix de l'élasticité.

En vue de la réalisation de ce but, on prévoit selon l'invention un système d'ostéosynthèse pour colonne vertébrale, comportant au moins deux organes d'ancrage adaptés à être fixés à deux vertèbres de la colonne vertébrale, et un organe de liaison adapté à relier les deux organes d'ancrage en exerçant des contraintes à l'encontre du rapprochement des deux organes d'ancrage en translation l'un vers l'autre, l'organe de liaison étant déformable. élastiquement en flexion autour d'au moins un axe de déformation, l'organe de liaison comportant au moins deux portions de fixation adaptées à être fixées aux deux organes d'ancrage et au moins une portion intermédiaire telle que les deux portions de fixation sont reliées l'une à l'autre uniquement par la portion intermédiaire, la portion intermédiaire étant décalée par rapport à un axe d'alignement défini par les deux portions de fixation. Pour la ou chaque portion intermédiaire, l'organe de liaison comporte en outre deux butées s'étendant en regard l'une de l'autre et disposées de façon à venir en appui l'une contre l'autre lorsque l'organe de liaison est déformé autour de l'axe ou au moins l'un des axes de déformation dans un sens de rotation donné sur un angle égal à une valeur limite prédéterminée.

Ainsi, on limite l'amplitude de la déformation de l'organe de liaison dans ce sens de rotation. Au moyen des butées, on contrôle donc l'aptitude de l'organe de liaison à se déformer sans que cela influence le choix de l'élasticité de la portion intermédiaire.

Lorsque le système comporte trois organes d'ancrage avec deux des organes d'ancrage rigidement reliés l'un à l'autre et deux des organes d'ancrage reliés entre eux par l'organe de liaison déformable et se trouvant à une extrémité de la série, on constitue une transition ou gradient de rigidité entre la partie rigide de la colonne et sa partie libre. On obtient par conséquent une meilleure répartition des contraintes le long de la colonne. On évite l'apparition d'une discontinuité brutale dans la répartition des contraintes entre la dernière vertèbre de la série rigide et la première vertèbre libre, et on favorise au contraire une répartition harmonieuse des contraintes le long de la colonne. Par ailleurs, lorsque le système ne concerne que deux vertèbres, on réalise une arthrodèse souple entre ces deux vertèbres.

Avantageusement, l'axe ou au moins l'un des axes de déformation s'étend perpendiculairement à une direction longitudinale de chaque organe d'ancrage et à une direction longitudinale de l'organe de liaison.

Avantageusement, l'axe ou au moins l'un des axes de déformation s'étend sensiblement parallèlement à une direction longitudinale de chaque organe d'ancrage.

Avantageusement, l'axe ou au moins l'un des axes de déformation s'étend parallèlement à une direction longitudinale de l'organe de liaison.

Ainsi, chacun de ces axes correspond à un axe de déplacement relatif en rotation des deux organes d'ancrage l'un par rapport à l'autre, en raison du mouvement des vertèbres. Suivant les cas, le disque intervertébral est donc soulagé au moins partiellement des contraintes correspondant à ces déplacements respectifs.

Avantageusement, l'axe d'alignement s'étend entre la portion intermédiaire d'une part et les deux butées d'autre part.

Avantageusement, la portion intermédiaire présente une face externe de forme sphérique.

Avantageusement, les deux butées présentent chacune une face externe de forme sphérique.

Ainsi, dans ces deux cas, le système étant destiné à se trouver à l'intérieur du corps, on limite le nombre d'arêtes vives et on dispose les arêtes vives en réduisant les risques d'agression engendrés par la présence du système dans le corps du patient.

Avantageusement, la face sphérique de la portion intermédiaire et la face sphérique des deux butées ont un même centre de courbure.

Avantageusement, pour le ou chaque couple de portions de fixation, l'organe de liaison comporte une portion de jonction contiguë aux deux portions de fixation, la portion de jonction associée au couple ou à au moins l'un des couples de portions de fixation étant d'une seule pièce.

Avantageusement, pour le ou chaque couple de portions de fixation, l'organe de liaison comporte une portion de jonction contiguë aux deux portions de fixation, la portion de jonction associée au couple ou à au moins l'un des couples de portions de fixation comportant un premier et un deuxième éléments de liaison distincts l'un de l'autre, et des moyens de fixation des premier et deuxième éléments de liaison l'un à l'autre.

Ainsi, l'organe de liaison sera d'autant plus facile à fabriquer que la partie de jonction sera en deux pièces ou que l'organe de liaison comportera un grand nombre de parties de jonction en deux pièces par rapport au nombre total de parties de jonction.

Avantageusement, ces moyens de fixation sont adaptés à permettre de régler la distance entre les deux organes d'ancrage associés, suivant la direction longitudinale de l'organe de liaison.

Ainsi, la longueur de l'organe de liaison est réglable. Lors de l'installation du système, on peut donc adapter la distance entre les deux organes d'ancrage à la position souhaitée pour les deux vertèbres associées à ces organes d'ancrage.

Avantageusement, le deuxième élément de liaison comporte une tige et le premier élément de liaison présente un logement adapté à recevoir la tige, le premier élément de liaison comportant des moyens de blocage de la tige à l'intérieur du logement dans plusieurs positions suivant la direction longitudinale de l'organe de liaison.

Avantageusement, le système est adapté pour que l'axe d'alignement s'étende entre la portion intermédiaire et les vertèbres.

Ainsi, lorsque les deux vertèbres sont soumises à des contraintes tendant à rapprocher l'un de l'autre les deux organes d'ancrage en translation suivant l'axe d'alignement, cet agencement induit une augmentation du bras de levier intervenant dans le moment de flexion lors de la flexion suivant l'un des axes de déformation. La portion intermédiaire est donc soumise à un moment de flexion plus important et subit une déformation élastique accrue. Ceci permet d'augmenter la capacité de flexion du segment rachidien arthrodésé autour de cet axe et de créer une meilleure transition des contraintes avec les étages non fixés.

D'autres caractéristiques et avantages de l'invention apparaîtront encore dans la description qui va suivre de deux modes de réalisation préférés donnés à titre d'exemples non limitatifs. Aux dessins annexés:
- la figure 1 est une vue en élévation d'un premier mode de réalisation du système d'ostéosynthèse selon l'invention fixé à deux vertèbres;
- la figure 2 est une vue en coupe transversale suivant le plan II-II de l'organe de liaison de la figure 1;
- la figure 3 est une vue partielle en élévation d'un deuxième mode de réalisation du système d'ostéosynthèse selon l'invention fixé à des vertèbres; et
- la figure 4 est une vue en élévation partiellement en coupe axiale de l'organe de liaison de la figure 3.

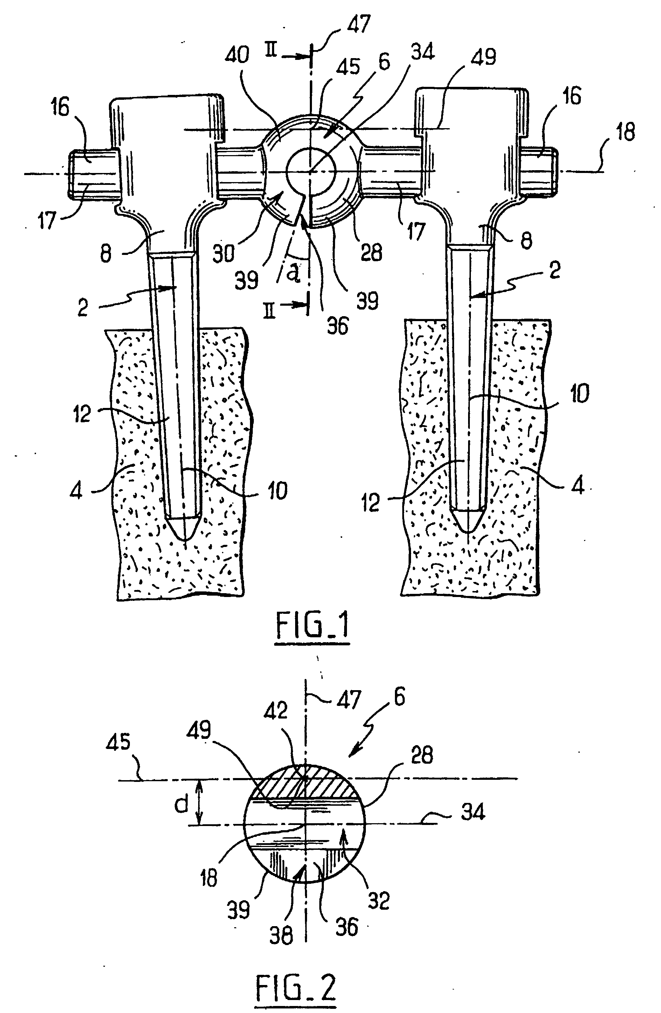

En référence aux figures 1 à 2, un premier mode de réalisation du système d'ostéosynthèse selon l'invention pour colonne vertébrale permet de constituer le dispositif d'ostéosynthèse représenté. Le système comporte seulement un premier et un deuxième organes d'ancrage ou vis pédiculaires 2 adaptés à être fixés respectivement à deux vertèbres 4 adjacentes l'une de l'autre de la colonne vertébrale d'un patient. Le système comporte en outre un organe de liaison 6 adapté à relier ces deux organes d'ancrage 2 entre eux.

Chacun des deux organes d'ancrage 2 comporte un corps allongé 8 d'axe longitudinal 10 présentant un pied fileté 12 de forme légèrement conique d'axe 10 se rétrécissant vers l'extrémité libre du pied, adapté à être ancré dans la vertèbre associée 4. Le corps 10 comporte une tête opposée longitudinalement à l'extrémité libre du pied 12 et destinée à s'étendre à l'extérieur de la vertèbre 4 dans le corps du patient.

L'organe de liaison 6 est ici d'une seule pièce. Il comporte un première et une deuxième portions de fixation 16 comprenant chacune une tige cylindrique rectiligne 17. Les deux tiges 17 sont alignées l'une avec l'autre et présentent un axe longitudinal commun 18 qui est l'axe longitudinal de l'organe de liaison et l'axe d'alignement des deux tiges 17.

Chaque portion de fixation 16 est adaptée à être fixée rigidement à la tête de l'un des organes d'ancrage selon des moyens connus. L'axe 18 de l'organe de liaison est alors sensiblement perpendiculaire à l'axe 10 de l'organ d'ancrage.

Lors de l'installation du système d'ostéosynthèse, on ancre chaque organe d'ancrage 2 dans la vertèbre 4 associée, puis on fixe les organes d'ancrage 2 rigidement à l'organe de liaison 6.

L'organe de liaison 6 comporte en outre une partie de jonction 28 contiguë aux deux portions de fixation 16, par une extrémité de chaque tige 17 et s'étendant dans l'axe longitudinal 18 entre les deux tiges. La partie de jonction 28 présente une face externe 30 de forme générale sphérique, ayant un centre de courbure situé sur l'axe longitudinal 18, à mi-distance des deux portions d'extrémité 16.

La partie de jonction 28 présente un évidement cylindrique 32 la traversant de part en part suivant un diamètre de la sphère. L'évidement cylindrique 32 a un axe de cylindre 34 interceptant l'axe longitudinal 18 de l'organe de liaison à mi-distance des deux portions d'extrémité 16 et s'étendant perpendiculairement à cet axe longitudinal 18.

La partie de jonction 28 présente en outre une échancrure 36 en forme de quartier de spbèxe, délimitée par deux faces planes 38 de la partie de jonction s'étendant en regard l'une de l'autre et passant chacune par l'axe 34 de l'évidement cylindrique. L'échancrure 36 s'étend suivant une direction radiale, à l'axe de cylindre 34, depuis la face externe sphérique 30 jusqu'à l'évidement cylindrique 32. L'angle a de cette échancrure, défini autour de l'axe 34 de l'évidement cylindrique par les deux faces planes 38, est environ de 15°. L'une des faces planes 38 est perpendiculaire à l'axe longitudinal 18 de sorte que l'échancrure s'étend d'un côté du plan médian transversal à l'axe 18 de l'organe de liaison.

L'évidement cylindrique 34 et l'échancrure 36 permettent de distinguer deux portions de la partie de jonction 28 s'étendant de part et d'autre de l'axe longitudinal et d'alignement 18. Ainsi, la partie de jonction comporte deux butées 39 s'étendant d'un premier côté de l'axe longitudinal 18 associé à l'échancrure 36. Chaque butée 39 s'étend depuis l'une des portions de fixation respectives 16 jusqu'à l'échancrure 36, les faces 38 en regard constituant deux faces d'extrémité des butées. La partie de jonction 28 comporte également une portion intermédiaire 40 s'étendant d'un deuxième côté de l'axe longitudinal 18 opposé à l'échancrure 36.

Ainsi, les deux butées 39 s'étendent d'un même côté de la portion intermédiaire 40 que l'axe longitudinal 18. Cet axe 18 s'étend entre la portion intermédiaire 40 d'une part et les deux butées 39 d'autre part.

La face sphérique 30 constitue une face externe des butées 39 et de la portion intermédiaire 40. Les faces sphériques de la portion intermédiaire et des butées ont un même centre de courbure. L'évidement cylindrique 32 définit une face interne cylindrique des butées 38 et de la portion intermédiaire 40.

La portion intermédiaire 40 est décalée latéralement par rapport à l'axe longitudinal 18 qui est l'axe d'alignement des deux portions de fixation 16. La portion intermédiaire 40 présente une fibre neutre 42. Le décalage présente une mesure "d" prise entre la fibre neutre 42 et l'axe longitudinal 18 et s'étendant perpendiculairement à cet axe. Cette mesure est ici égale à environ le diamètre des tiges 17 des portions d'extrémité 16.

Les deux portions d'extrémité 16 sont reliées l'une à l'autre seulement par la portion intermédiaire 40. Celle-ci exerce des contraintes s'opposant au rapprochement et à l'éloignement en translation suivant l'axe longitudinal 18 des deux portions de fixations 16 l'une de l'autre, et donc des deux organes d'ancrage 2 l'un de l'autre. La portion intermédiaire 40 est déformable élastiquement autour d'un premier axe de déformation 45 parallèle à l'axe 34 de l'évidement 32 et traversant la portion intermédiaire au niveau de la fibre neutre 42.

Elle l'est également autour d'un deuxième axe de déformation 47 perpendiculaire à l'axe longitudinal 18 et au premier axe 45, passant par le centre de la sphère et interceptant les axes 45 et 18. Elle l'est encore autour d'un troisième axe de déformation 49 parallèle à l'axe longitudinal 18 et perpendiculaire au premier axe 45 en interceptant celui-ci à la fibre neutre de la portion intermédiaire. Les deuxième et troisième axes 47 et 49 traversent la portion intermédiaire 40. Les trois axes 45, 47, 49 sont perpendiculaires entre eux, se coupent en un même point et forment un trièdre.

Lors de l'installation du système, on oriente l'organe de liaison 6 autour de son axe longitudinal 18 de sorte que les butées 39 se trouvent du côté des vertèbres 4, la portion intermédiaire 40 se trouvant du côté opposé aux vertèbres 4. Ainsi, le premier axe de déformation 45 s'étend perpendiculairement à l'axe longitudinal 10 des organes d'ancrage 2 et le deuxième axe de déformation 47 est sensiblement parallèle aux axes 10. La portion intermédiaire 40 est adaptée à se fléchir suivant chacun des trois axes de déformation pour encaisser au moins une partie des contraintes s'exerçant normalement sur le disque situé entre les deux vertèbres 4. Cette flexion s'effectue suivant un seul ou plusieurs de ces axes à la fois.

Les deux butées 39 viennent en appui l'une contre l'autre lorsque la portion intermédiaire 40 est fléchie autour du premier axe de déformation 45, sur un angle a égal à une valeur limite prédéterminée, liée à la forme du corps 28, dans le sens de rotation correspondant à un rapprochement des deux butées l'une de l'autre. On interdit ainsi la déformation de l'organe de liaison sur un angle supérieur à la valeur prédéterminée.

Ce système réalise une arthrodèse souple entre les deux vertèbres.

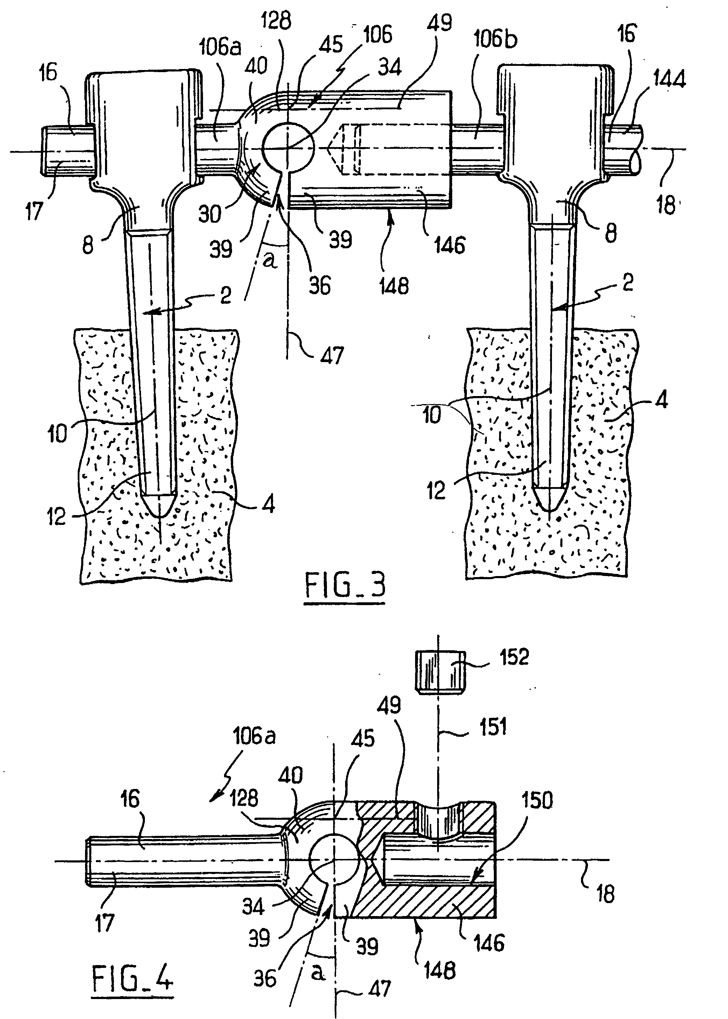

Les figures 3 et 4 représentent un deuxième mode de réalisation du système d'ostéosynthèse selon l'invention dans lequel les éléments différents portent des références augmentées de cent.

Le système comporte en outre cette fois un troisième organe d'ancrage, non représenté, et s'étendant à droite du dispositif visible sur la figure 3. Les trois organes d'ancrage sont chacun identiques à ceux du premier mode. L'organe de liaison 106 comporte une partie de jonction 128 comprenant cette fois un premier élément de liaison 106a et un deuxième élément de liaison 106b distincts l'un de l'autre.

Le deuxième élément de liaison 106b comporte une tige rigide allongée rectiligne 144 d'axe longitudinal 18 et à section circulaire. La fixation au deuxième élément de liaison 106b des deuxième et troisième organes d'ancrage s'effectue de la même façon que dans le premier mode. Le deuxième élément de liaison 106b comporte notamment la deuxième portion de fixation 16.

Le premier élément de liaison 106a comporte une première portion de fixation 16 identique à la première portion de fixation du premier mode. Il comporte également, contiguës à une extrémité de la portion de fixation, une portion intermédiaire 40 et deux butées 39. Le premier élément de liaison 106a comporte en outre un manchon cylindrique 146 présentant une face externe cylindrique 148 de même rayon que le rayon de courbure de la face sphérique 30 et une face interne cylindrique 150. La portion de fixation 16 et le manchon 146 ont pour axe l'axe longitudinal 18 de l'organe de liaison. La face externe 148 s'étend dans le prolongement et la continuité de la face sphérique 30 à l'extrémité opposée longitudinalement à la portion de fixation 16. Ainsi, la face externe de l'une des butées 39 et une partie de la face externe de la portion intermédiaire 40 ont une forme cylindrique. La face interne 150 du manchon 146 a un rayon légèrement supérieur au rayon de la tige 144 du deuxième élément 106b. Cette face interne 150 définit un logement adapté à recevoir cette tige.

La paroi du manchon 146 est traversée par un conduit cylindrique d'axe 151 perpendiculaire à l'axe longitudinal 18 du manchon et débouchant sur les faces externe 148 et interne 150 du manchon. Ce conduit 151 est fileté, le deuxième élément de liaison 106b comportant une vis 152 adaptée à être vissée dans ce conduit pour venir en butée contre la tige 144 reçue dans le logement 150 en vue de bloquer la tige dans le logement dans plusieurs positions au choix suivant l'axe longitudinal 18. La tige 144, le logement 150 et la vis 152 constituent ainsi des moyens de fixation des deux éléments de liaison 106a et 106b l'un à l'autre, permettant de régler la distance entre les deux portions de fixation 16 suivant l'axe longitudinal 18.

Pour l'installation du dispositif d'ostéosynthèse, on fixe les trois organes d'ancrage aux vertèbres 4, on engage la tige 144 du deuxième élément 106b dans le logement 150 du premier élément de liaison 106a sans serrer la vis 152 de sorte que la tige 144 demeure libre de coulisser dans le logement suivant l'axe longitudinal 18. On met en place l'organe de liaison 106 et on fixe chacun des trois organes d'ancrage à l'organe de liaison 106. On choisit ensuite la position de la tige 144 dans le logement 150 en fonction de la position désirée suivant l'axe longitudinal 18 pour la vertèbre 4 adjacente associée au premier élément de liaison 106a par rapport à la vertèbre 4 associée au deuxième élément de liaison 106b. On immobilise ensuite ces deux éléments de liaison relativement par serrage de la vis 152 contre la tige 144.

La tige 144 constitue une partie de l'organe de liaison 106 adaptée à relier rigidement le troisième organe d'ancrage au deuxième organe d'ancrage. Les trois organes d'ancrage sont reliés entre eux par l'organe de liaison 106.

Les différentes pièces du système d'ostéosynthèse seront par exemple en alliage biocompatible.

Dans chacun de ces deux modes, la colonne vertébrale du patient sera munie de deux systèmes d'ostéosynthèse disposés symétriquement de part et d'autre d'un plan longitudinal médian de la colonne en étant fixés aux mêmes vertèbres.

L'amplitude maximale des flexions sera typiquement de 2° autour du troisième axe de déformation 49 (torsion) et de 5 à 6° autour du deuxième axe de déformation 47 (flexion latérale).

Bien entendu, on pourra apporter à l'invention de nombreuses modifications sans sortir du cadre de celle-ci. L'organe de liaison 6 du premier mode de réalisation, de longueur fixe, pourra être adapté pour être associé à au moins trois organes d'ancrage. Inversement, l'organe de liaison 106 du deuxième mode de réalisation, de longueur variable, pourra être adapté pour être associé à seulement deux organes d'ancrage.

Ce système pourra comporter plus de trois organes d'ancrage. Un même organe de liaison pourra notamment comporter une ou plusieurs parties de jonction 28 (d'une seule pièce) et/ou une ou plusieurs parties de jonction 128 en deux éléments distincts, chaque partie de jonction étant destinée à s'étendre entre deux organes d'ancrage. Avantageusement, l'organe de liaison comportera une partie de jonction 28 d'une seule pièce à une extrémité, suivie de plusieurs parties de jonction 128 à deux éléments.

Le centre de courbure de la face sphérique de la portion intermédiaire et/ou le centre de courbure de la face sphérique des butées pourra être situé ailleurs que sur l'axe 34 de l'évidement cylindrique 32.

Indépendamment de la présence ou de l'absence des butées, on pourra prévoir plus généralement un système d'ostéosynthèse pour colonne vertébrale, comportant au moins des premier, deuxième et troisième organes d'ancrage adaptés à être fixés à des première, deuxième et troisième vertèbres respectives de la colonne vertébrale, et un organe de liaison (en une ou plusieurs pièces) adapté à relier rigidement l'un à l'autre les deuxième et troisième organes d'ancrage, et adapté à relier l'un à l'autre les premier et deuxième organes d'ancrage-en exerçant des contraintes à l'encontre du rapprochement des premier et deuxième organes d'ancrage en translation l'un vers l'autre et en étant déformable élastiquement en flexion autour d'au moins un axe de déformation entre les premier et deuxième organes d'ancrage.

Ainsi, lorsque les première et deuxième vertèbres sont soumises à des contraintes tendant à déplacer relativement les premier et deuxième organes d'ancrage en rotation autour de l'axe de déformation, l'organe de liaison est soumis à un moment de flexion et se fléchit élastiquement autour de l'axe de déformation. Il encaisse donc partiellement ces contraintes. Le système recrée partiellement la biomécanique de l'unité fonctionnelle composée des première et deuxième vertèbres fixées, ainsi que du disque intervertébral et permet ainsi d'atténuer le report des contraintes sur le disque susjacent à la fixation. Le système comportant trois organes d'ancrage avec deux des organes d'ancrage rigidement reliés l'un à l'autre et deux des organes d'ancrage reliés entre eux par la partie déformable de l'organe de liaison et se trouvant à une extrémité de la série, on constitue une transition ou gradient de rigidité entre la partie rigide de la colonne et sa partie libre. On obtient par conséquent une meilleure répartition des contraintes le long de la colonne.

Un tel système d'ostéosynthèse évite, lorsque le système concerne au moins trois vertèbres reliées entre elles, l'apparition d'une discontinuité brutale dans la répartition des contraintes entre la dernière vertèbre de la série rigide et la première vertèbre libre, et favorise au contraire une répartition harmonieuse des contraintes le long de la colonne.

## Revendications

1. Système d'ostéosynthèse pour colonne vertébrale, comportant au moins deux organes d'ancrage (2) adaptés à être fixés à deux vertèbres (4) de la colonne vertébrale, et un organe de liaison (6; 106) adapté à relier les deux organes d'ancrage en exerçant des contraintes à l'encontre du rapprochement des deux organes d'ancrage en translation l'un vers l'autre, l'organe de liaison (6; 106) étant déformable élastiquement en flexion autour d'au moins un axe de déformation (45, 47, 49), l'organe de liaison (6; 106) comportant au moins deux portions de fixation (16) adaptées à être fixées aux deux organes d'ancrage (2) et au moins une portion intermédiaire (40) telle que les deux portions de fixation sont reliées l'une à l'autre uniquement par la portion intermédiaire, la portion intermédiaire étant décalée par rapport à un axe d'alignement (18) défini par les deux portions de fixation (16), **caractérisé en ce que** pour la ou chaque portion intermédiaire (40), l'organe de liaison (6; 106) comporte en outre deux butées (39) s'étendant en regard l'une de l'autre et disposées de façon à venir en appui l'une contre l'autre lorsque l'organe de liaison est déformé autour de l'axe ou au moins l'un (45) des axes de déformation dans un sens de rotation donné sur un angle (a) égal à une valeur limite prédéterminée.

2. Système selon la revendication 1, **caractérisé en ce que** l'axe ou au moins l'un (45) des axes de déformation s'étend perpendiculairement à une direction longitudinale (10) de chaque organe d'ancrage (2) et à une direction longitudinale (18) de l'organe de liaison (6 ; 106).

3. Système selon la revendication 1 ou 2, **caractérisé en ce que** l'axe ou au moins l'un (47) des axes de déformation s'étend sensiblement parallèlement à une direction longitudinale (10) de chaque organe d'ancrage (2).

4. Système selon l'une quelconque des revendications 1 à 3, **caractérisé en ce que** l'axe ou au moins l'un (49) des axes de déformation s'étend parallèlement à une direction longitudinale (18) de l'organe de liaison (6 ; 106).

5. Système selon l'une quelconque des revendications 1 à 4, **caractérisé en ce que** l'axe d'alignement (18) s'étend entre la portion intermédiaire (40) d'une part et les deux butées (39) d'autre part.

6. Système selon l'une quelconque des revendications 1 à 5, **caractérisé en ce que** la portion intermédiaire (40) présente une face externe (30) de forme sphérique.

7. Système selon l'une quelconque des revendications 1 à 6, **caractérisé en ce que** les deux butées (39) présentent chacune une face externe (30) de forme sphérique.

8. Système selon les revendications 6 et 7 **caractérisé en ce que** la face sphérique (30) de la portion intermédiaire et la face sphérique (30) des deux butées ont un même centre de courbure.

9. Système selon l'une quelconque des revendications 1 à 8, **caractérisé en ce que**, pour le ou chaque couple de portions de fixation (16), l'organe de liaison (16) comporte une portion de jonction (28) contiguë aux deux portions de fixation, la portion de jonction associée au couple ou à au moins l'un des couples de portions de fixation étant d'une seule pièce.

10. système selon l'une quelconque des revendications 1 à 9, **caractérisé en ce que**, pour le ou chaque couple de portions de fixation (16), l'organe de liaison (106) comporte une portion de jonction (128) contiguë aux deux portions de fixation, la portion de jonction associée au couple ou à au moins l'un des couples de portions de fixation comportant un premier et un deuxième éléments de liaison (106a, 106b) distincts l'un de l'autre, et des moyens de fixation des premier et deuxième éléments de liaison l'un à l'autre.

11. Système selon la revendication 10, **caractérisé en ce que** ces moyens de fixation sont adaptés à permettre de régler la distance entre les deux organes d'ancrage (2) associés, suivant la direction longitudinale (18) de l'organe de liaison (106).

12. Système selon la revendication 11, **caractérisé en ce que** le deuxième élément de liaison (106b) comporte une tige (144) et le premier élément de liaison (106a) présente un logement (150) adapté à recevoir la tige, le premier élément de liaison (106b) comportant des moyens de blocage (152) de la tige à l'intérieur du logement (150) dans plusieurs positions suivant la direction longitudinale (18) de l'organe de liaison.

13. Système selon l'une quelconque des revendications 1 à 12, **caractérisé en ce qu'**il est adapté pour que l'axe d'alignement (18) s'étende entre la portion intermédiaire (40) et les vertèbres (4).

## Claims

1. An osteosynthesis system for the spinal column, the system comprising at least two anchor members (2) adapted to be fixed to two vertebrae (4) of the spinal column, and a link member (6; 106) adapted to interconnect the two anchor members and exerting stresses against movement of the two anchor members in translation towards each other, the link member (6; 106) being elastically deformable in bending about at least one deformation axis (45, 47, 49), the link member (6; 106) having at least two fixing portions (16) adapted to be fixed to the two anchor members (2), and at least one intermediate portion (40) such that the two fixing portions are connected to each other solely by the intermediate portion, the intermediate portion being offset from an alignment axis (18) defined by the two fixing portions (16), the system being **characterized in that** for the or each intermediate portion (40) the link member (6; 106) further has two abutments (39) facing each other and disposed so as to come into abutment against each other when the link member is deformed about the deformation axis, or at least one (45) of the deformation axes, in a given direction of rotation and through an angle (a) equal to a predetermined limit value.

2. A system according to claim 1, **characterized in that** the deformation axis or at least one (45) of the deformation axes extends perpendicularly to a longitudinal direction (10) of each anchor member (2) and to a longitudinal direction (18) of the link member (6; 106).

3. A system according to claim 1 or 2, **characterized in that** the deformation axis or at least one (47) of the deformation axes extends substantially parallel to a longitudinal direction (10) of each anchor member (2).

4. A system according to any one of claims 1 to 3, **characterized in that** the deformation axis or at least one (49) of the deformation axes extends parallel to a longitudinal direction (18) of the link member (6; 106).

5. A system according to any one of claims 1 to 4, **characterized in that** the alignment axis (18) lies between the intermediate portion (40) and the two abutments (39).

6. A system according to any one of claims 1 to 5, **characterized in that** the intermediate portion (40) has an outer face (30) of spherical shape.

7. A system according to any one of claims 1 to 6, **characterized in that** each of the two abutments (39) has an outer face (30) of spherical shape.

8. A system according to claims 6 and 7, **characterized in that** the spherical face (30) of the intermediate portion and the spherical faces (30) of the two abutments have a common center of curvature.

9. A system according to any one of claims 1 to 8, **characterized in that** for the or each pair of fixing portions (16), the link member (16) has a junction part (28) contiguous with the two fixing portions, the junction part associated with the or at least one of the pairs of fixing portions being a single piece.

10. A system according to any one of claims 1 to 9, **characterized in that** for the or each pair of fixing portions (16) the link member (106) has a junction part (128) contiguous with the two fixing portions, the junction part associated with the or at least one of the pairs of fixing portions having first and second mutually distinct link elements (106a, 106b) and fixing means for fixing the first and second link elements together.

11. A system according to claim 10, **characterized in that** the fixing means are adapted to enable the distance between the two associated anchor members (2) to be adjusted in the longitudinal direction (18) of the link member (106).

12. A system according to claim 11, **characterized in that** the second link element (106b) comprises a rod (144) and the first link element (106a) has a housing (150) suitable for receiving the rod, the first link element (106a) including locking means (152) for locking the rod inside the housing (150) in a plurality of positions along the longitudinal direction (18) of the link member.

13. A system according to any one of claims 1 to 12, **characterized in that** it is adapted so that the alignment axis (18) lies between the intermediate portion (40) and the vertebrae (4).

## Patentansprüche

1. Osteosynthesesystem für die Wirbelsäule, mit mindestens zwei Verankerungsorganen (2), die dazu eingerichtet sind, an zwei Wirbeln (4) der Wirbelsäule befestigt zu werden, und einem Verbindungsorgan (6; 106), das dazu eingerichtet ist, die beiden Verankerungsorgane zu verbinden, indem es Kräfte gegen die Annäherung der beiden Verankerungsorgane bei der Translation zueinander ausübt, wobei das Verbindungsorgan (6; 106) elastisch um mindestens eine Verformungsachse (45, 47, 49) biegeverformbar ist, wobei das Verbindungsorgan (6; 106) mindestens zwei Befestigungsabschnitte (16), die dazu eingerichtet sind, an den beiden Verankerungsorganen (2) befestigt zu werden, und mindestens einen Zwischenabschnitt (40) aufweist, so daß die beiden Befestigungsabschnitte alleine über den Zwischenabschnitt verbunden sind, und wobei der Zwischenabschnitt bezüglich einer Ausrichtachse (18), die durch die beiden Befestigungsabschnitte (16) definiert ist, versetzt ist, **dadurch gekennzeichnet, daß**, für den oder jeden Zwischenabschnitt (40), das Verbindungsorgan (6; 106) außerdem zwei Anschläge (39) aufweist, die sich einander gegenüberliegend erstrecken und derart angeordnet sind, daß sie in gegenseitige Anlage gelangen, wenn das Verbindungsorgan rund um die Verformungsachse oder mindestens die eine (45) der Verformungsachsen in einer gegebenen Drehrichtung um einen Winkel (a) verformt ist, der einem bestimmten Grenzwert entspricht.

2. System nach Anspruch 1, **dadurch gekennzeichnet, daß** die Verformungsachse oder mindestens die eine (45) der Verformungsachsen sich senkrecht zu einer Längsrichtung (10) eines jeden Verankerungsorganes (2) und zu einer Längsrichtung (18) des Verbindungsorganes (6; 106) erstreckt.

3. System nach Anspruch 1 oder 2, **dadurch gekennzeichnet, daß** die Verformungsachse oder mindestens die eine (47) der Verformungsachsen sich im wesentlichen parallel zu einer Längsrichtung (10) eines jeden Verankerungsorganes (2) erstreckt.

4. System nach irgendeinem der Ansprüche 1 bis 3, **dadurch gekennzeichnet, daß** die Verformungsachse oder mindestens die eine (49) der Verformungsachsen sich parallel zu einer Längsrichtung (18) des Verbindungsorganes (6; 106) erstreckt.

5. System nach irgendeinem der Ansprüche 1 bis 4, **dadurch gekennzeichnet, daß** die Ausrichtachse (18) sich zwischen einerseits dem Zwischenabschnitt (40) und andererseits den beiden Anschlägen (39) erstreckt.

6. System nach irgendeinem der Ansprüche 1 bis 5, **dadurch gekennzeichnet, daß** der Zwischenabschnitt (40) eine Außenfläche (30) mit kugeliger Form aufweist.

7. System nach irgendeinem der Ansprüche 1 bis 6, **dadurch gekennzeichnet, daß** die beiden Anschläge (39) jeweils eine Außenfläche (30) mit kugeliger Form aufweisen.

8. System nach den Ansprüchen 6 und 7, **dadurch gekennzeichnet, daß** die kugelige Fläche (30) des Zwischenabschnitts und die kugelige Fläche (30) der beiden Anschläge ein und dieselbe-Krümmungsmitte aufweisen.

9. System nach irgendeinem der Ansprüche 1 bis 8, **dadurch gekennzeichnet, daß** für das oder für jedes Paar von Befestigungsabschnitten (16) das Verbindungsorgan (16) einen Zusammenfügungsabschnitt (28) aufweist, der an die beiden Befestigungsabschnitte angrenzt, wobei der Zusammenfügungsabschnitt, der dem Paar oder mindestens einem der Paare von Befestigungsabschnitten zugeordnet ist, einstückig ist.

10. System nach irgendeinem der Ansprüche 1 bis 9, **dadurch gekennzeichnet, daß** für das oder für jedes Paar von Betestigungsabschnitten (16) das Verbindungsorgan (106) einen Zusammenfügungsabschnitt (128) aufweist, der an die beiden Befestigungsabschnitte angrenzt, wobei der Zusammenfügungsabschnitt, der dem Paar oder mindestens einem der Paare von Befestigungsabschnitten zugeordnet ist, ein erstes und ein zweites Verbindungselement (106a, 106b), die voneinander unterschieden sind, sowie Mittel zur Befestigung des ersten und zweiten Verbindungselements aneinander aufweist.

11. System nach Anspruch 10, **dadurch gekennzeichnet, daß** diese Befestigungsmittel dazu eingerichtet sind, die Einregulierung des Abstandes zwischen den beiden, zugeordneten Verankerungsorganen (2) entsprechend der Längsrichtung (18) des Verbindungsorganes (106) zu gestatten.

12. System nach Anspruch 11, **dadurch gekennzeichnet, daß** das zweite Verbindungselement (106b) eine Stange (144) aufweist und das erste Verbindungselement (106a) einen Sitz (150) darbietet, der zur Aufnahme der Stange eingerichtet ist, wobei das erste Verbindungselement (106b) Blockierungsmittel (152) für die Stange im Inneren des Sitzes (150) in mehreren Lagen entsprechend der Längsrichtung (18) des Verbindungsorganes aufweist.

13. System nach irgendeinem der Ansprüche 1 bis 12, **dadurch gekennzeichnet, daß** die Ausrichtachse (18) sich zwischen dem Zwischenabschnitt (40) und den Wirbeln (4) erstreckt.
